# EUROPÄISCHE PATENTSCHRIFT

(11) **EP 3 378 499 B1**
(45) Veröffentlichungstag und Bekanntmachung des Hinweises auf die Patenterteilung: **01.09.2021**
(21) Anmeldenummer: 18162490.9
(22) Anmeldetag: 19.03.2018
(51) Int. Cl.: A61L 2/18, B01F 15/00

(54) **VORRICHTUNG UND VERFAHREN ZUM DOSIEREN VON DESINFEKTIONS- UND/ODER REINIGUNGSMITTELN**
DEVICE AND METHOD FOR DOSING OF DISINFECTANTS AND/OR CLEANING AGENTS
DISPOSITIF ET PROCÉDÉ DE DISTRIBUTION D'AGENTS DE DÉSINFECTION ET/OU DE NETTOYAGE

(30) Priorität: 20.03.2017 DE 102017105907; 24.05.2017 DE 102017111332
(43) Veröffentlichungstag der Anmeldung: 26.09.2018
(73) Patentinhaber: Schulz GmbH, 58540 Meinerzhagen (DE)
(72) Erfinder: Schulz, Hartmut, 58540 Meinerzhagen (DE)
(74) Vertreter: Patentanwälte Dörner & Kötter PartG mbB

(56) Entgegenhaltungen:
- WO-A1-2005/011759
- WO-A2-2008/015678
- DE-A1- 10 342 952
- US-A- 5 251 825

## Beschreibung

Die Erfindung betrifft eine Vorrichtung zum Dosieren von Desinfektions- und/oder Reinigungsmitteln zur Erstellung von Gebrauchslösungen umfassend einen Wasserzulauf, ein Magnetventil, einen Durchflussmengensensor, einen Wasservorlaufbehälter sowie einen Auslauf. Die Erfindung betrifft außerdem ein Verfahren zum Dosieren von Desinfektions- und/oder Reinigungsmitteln zur Erstellung von Gebrauchslösungen.

Vorrichtungen zum Dosieren von Flüssigkeiten sind in vielfältiger Weise bekannt (vgl. bspw. US 4 738 541 A). Aus der WO 2005/011759 A1 ist zudem ein Gerät zur Erzeugung von wässrigem Chlordioxid durch Reaktion eines ersten Reagenz und eines zweiten Reagenz bekannt. Das Gerät weist zwei Pumpen sowie eine Reaktionskammer auf, wobei jeder Flüssigkeitsanteil im Wesentlichen mit konstantem Druck gepumpt wird.

An die Zubereitung von Desinfektionsmittelgebrauchslösungen und Reinigungsmittelgebrauchslösungen aus flüssigen Konzentraten in Verbindung mit Trinkwassernetzen werden jedoch erhöhte Anforderungen gestellt, beispielsweise Maßnahmen zur Verhinderung einer Verunreinigung des Trinkwassernetzes. Insbesondere muss jeder Rückfluss verhindert werden. Ein diesen Anforderungen entsprechendes Dosiergerät zur dezentralen Herstellung von Desinfektions- und/oder Reinigungsmittelgebrauchslösungen ist aus der DE 103 42 952 A1 bekannt.

Die Herstellung von Desinfektions- oder Reinigungsmittelgebrauchslösungen erfolgt zur Zeit fast ausschließlich unter Verwendung von flüssigen Desinfektions- und Reinigungsmittelkonzentraten. Diese flüssigen Konzentrate werden jedoch zunehmend, auch aufgrund gesetzlicher Änderungen, durch pulverförmige Konzentrate ersetzt. Dies hat seine Ursache darin, dass die Konzentrate überwiegend Aldehyde beinhalten. Diese sind unerwünscht, da Aldehyde irritierend auf Haut und Schleimhäuten wirken und Allergien auslösen können. Die Verwendung von aldehydfreien flüssigen Desinfektions- und Reinigungsmittelkonzentraten beinhaltet dabei den Nachteil, dass diese Konzentrate in der Regel nicht virozid wirken. Der Ersatz durch Peer-Essig-Säure ist kaum praktikabel, da die Behälter zur Aufbewahrung der Säure immer luftdicht bzw. dämpfedicht gelagert werden müssen, da die austretenden Dämpfe gesundheitsgefährdende Wirkung haben. Die Verwendung von Peer-Essig-Säure am Arbeitsplatz in ihrer Reinform ist nicht gestattet.

Vor dem Hintergrund des vorstehenden Sachverhalts kommen bereits Desinfektions- und Reinigungskonzentrate in Pulverform zur Anwendung, deren Anteil zukünftig aufgrund der genannten Umstände steigen wird. Problematisch bei der Verwendung der Pulver ist jedoch deren Anwendung. Die Pulver werden üblicherweise an den dezentral positionierten Dosiergeräten aufbewahrt. Für die Aufbewahrung sind diese üblicherweise in 1,5 kg-Gebinden abgepackt. Bei der Erstellung der Gebrauchslösungen ist das Personal genötigt, dass Pulver mithilfe eines Messlöffels in das Wasser zu geben. Dies hat nach genauen Vorgaben hinsichtlich der Menge an Pulver zu erfolgen.

Die manuelle Zugabe des Pulvers in das Wasser stellt eine erhebliche Verschlechterung bei der Herstellung der Gebrauchslösungen für das jeweilige Personal im Verhältnis zu den automatisch arbeitenden Dosiergeräten zur Verwendung flüssiger Konzentrate aus dem Stand der Technik dar. Diese Verschlechterung beruht zum einen in dem erheblich höheren Zeitaufwand, um das Pulver dem Wasser bei zu geben, da zunächst eine vorgegebene Menge Wasser in einen Behälter zu füllen ist, dem dann das Pulver manuell beizumischen ist. Zudem ergibt sich daraus eine inhomogene Verteilung des Pulvers im Wasser, so dass anschließend ein Rührvorgang erforderlich ist, um eine gleichmäßige Verteilung des Pulvers herbeizuführen. Der Zeitaufwand ist dadurch erheblich erhöht. Außerdem kann es durch die manuelle Beimengung des Pulvers zu Bedienfehlern kommen, so dass zu viel oder zu wenig Pulver dem Wasser beigemengt wird, wodurch die Wirksamkeit der Gebrauchslösung negativ beeinflusst ist.

Vor diesem Hintergrund ist es Aufgabe der vorliegenden Erfindung, eine Vorrichtung zum Dosieren von Desinfektions- und/oder Reinigungsmitteln zur Erstellung von Gebrauchslösungen zu schaffen, die eine automatische, dezentrale Beimengung von pulverförmigem Desinfektions- und/oder Reinigungsmittelkonzentrat ermöglicht. Gemäß der Erfindung wird diese Aufgabe mit den Merkmalen des Patentanspruchs 1 gelöst.

Mit der Erfindung ist eine Vorrichtung zum Dosieren von Desinfektions- und/oder Reinigungsmitteln zur Erstellung von Gebrauchslösungen geschaffen, welche die automatische Zugabe von Desinfektions- und/oder Reinigungsmitteln in Pulverform ermöglicht, ohne eine zentrale Lagerung des Pulvers zu erfordern. Vielmehr ist mit der erfindungsgemäßen Vorrichtung die von der Erstellung der Gebrauchslösungen aus flüssigen Konzentraten bekannten dezentralen Dosierung möglich. Folglich ist bei der Verwendung der erfindungsgemäßen Vorrichtung eine Änderung bzw. Erweiterung der Infrastruktur zur Erstellung der Gebrauchslösungen in den jeweiligen Pflegeeinrichtungen oder Krankenhäusern nicht erforderlich.

Die Aufgabe wird erfindungsgemäß darüber hinaus durch ein Verfahren zum Dosieren von pulverförmigen Desinfektions- und/oder Reinigungsmitteln zur Erstellung von Gebrauchslösungen mit den Merkmalen nach Patentanspruch 7 gelöst. Mit dem erfindungsgemäßen Verfahren ist ebenso wie mit der Vorrichtung eine dezentrale und automatische Erstellung von Gebrauchslösungen möglich. Mit dem erfindungsgemäßen Verfahren ist der Arbeitsaufwand für das Personal identisch zu demjenigen bei Verwendung von flüssigen Konzentraten.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die schematische Darstellung einer Vorrichtung zum Dosieren von Desinfektions- und/oder Reinigungsmitteln;
- Figur 2: die schematische Darstellung einer Vorrichtung zum Dosieren von Desinfektions- und/oder Reinigungsmitteln in anderer Ausgestaltung.

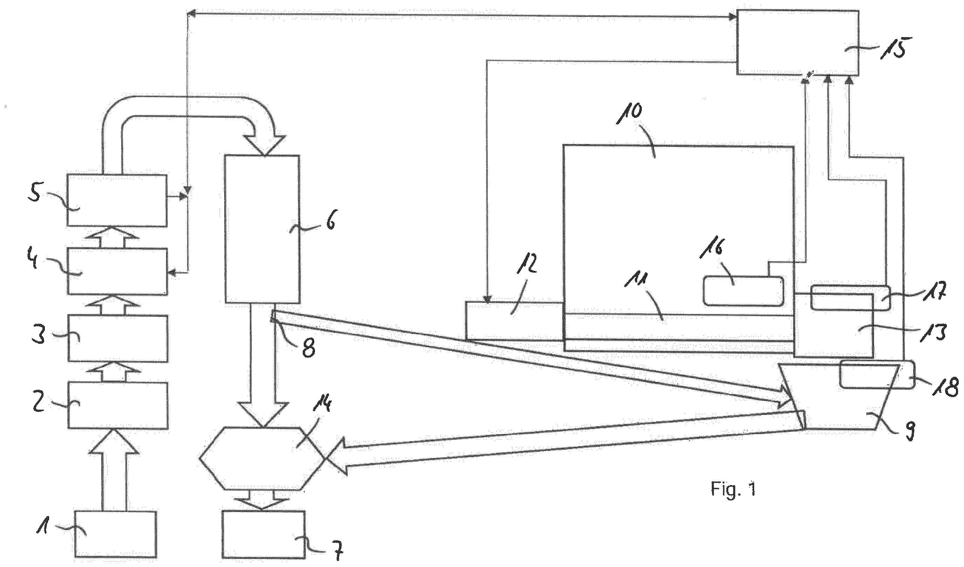

Die als Ausführungsbeispiel in Figur 1 gewählte Vorrichtung zum Dosieren von Desinfektions- und/oder Reinigungsmitteln nach der Erfindung besteht im Wesentlichen aus (Rohr-)Leitungen und Komponenten, die mittels der Leitungen miteinander in Verbindung stehen. Es ist ein Wasserzulauf 1 an ein Wasserversorgungsnetz angeschlossen. Der Wasserzulauf 1 ist über einen Schmutzfänger 2, bei dem es sich um ein Sieb handeln kann, einen Mengenregler 3 und ein Magnetventil 4 sowie einen Durchflussmengensensor 5 oder Flowmeter in einen Wasservorlaufbehälter 6 mit freiem Auslauf geführt. Der Wasservorlaufbehälter 6 hat die Funktion einer Systemtrennung; in ihr fällt das einlaufende Wasser über eine freie Strecke, so dass Einlauf und Auslauf getrennt sind.

Der Wasservorlaufbehälter 6 enthält eine - nicht dargestellte - Sonde für den Höchstfüllstand im Behälter. Steigt das Wasser in dem Behälter bis zum Erreichen der Sonde, spricht diese an und der Zulauf wird unterbrochen, um ein Überlaufen zu verhindern. Die Durchflussmenge ist bestimmt von der in dem Behälter 6 befindlichen Wassersäule über der Auslaufdüse. Da die Wassersäule innerhalb einer geringen Schwankungsbreite als konstant angesehen werden kann, ergibt sich in Abhängigkeit von der jeweils gewählten Auslaufdüse ein konstanter Durchfluss. Das Wasser verlässt die Vorrichtung zum Dosieren durch einen Auslauf 7, bei dem es sich beispielsweise um einen Schwenkauslauf handeln kann.

In Fließrichtung des Wassers hinter der Ausflussdüse ist ein Abzweig 8 vorgesehen. Der Abzweig 8 teilt das durchfließende Wasser in zwei Ströme auf: der erste Strom des Wassers fließt in Richtung des Auslaufs 7; der zweite Strom des Wassers fließt in Richtung einer Mischkammer 9, die mit dem Abzweig 8 über eine Leitung verbunden ist.

Die Mischkammer 9 ist Bestandteil einer Pulverdosiereinrichtung. Diese umfasst einen Vorratsbehälter 10 für ein Desinfektions- und/oder Reinigungspulver, bei dem es sich in der Regel um Konzentrat handelt. Der Vorratsbehälter 10 hat vorzugsweise in seinem dem Boden zugewandten Bereich einen V-förmigen Querschnitt. Er hat im Ausführungsbeispiel ein Fassungsvermögen von 1,5 kg Pulver. In dem Vorratsbehälter 10 ist bodenseitig eine Extruderschnecke 11 angeordnet, die von einem Motor 12 angetrieben ist. Der Motor 12 ist in zwei Richtungen betreibbar; der Motor 12 hat einen Vorwärts- und einen Rückwärtslauf. Dadurch ist die an den Motor 12 angebundene Extruderschnecke 11 in zwei Richtungen betreibbar: zum einen in Förderrichtung, in der Pulver aus dem Vorratsbehälter 10 herausgefördert werden kann, zum anderen in die entgegengesetzte Richtung, in der Pulver zurück in den Vorratsbehälter 10 gedrückt werden kann.

An der dem Motor 12 abgewandten Seite ist an dem Vorratsbehälter 10 ein Auslass 13 vorgesehen, der über der Mischkammer 9 positioniert ist. Die Mischkammer 9 ist mit einer Impfstelle 14 verbunden. Die Extruderschnecke 11 fördert das in dem Vorratsbehälter 10 befindliche Pulver zu dem Auslass 13, von dem aus das Pulver in die Mischammer 9 gelangt und dem zweiten Strom des Wassers beigemischt wird. Das Gemisch aus Wasser und Pulver gelangt von der Mischammer 9 zu einer Impfstelle 14, in der das Wasser-PulverGemisch dem ersten Strom des Wassers zugeführt wird und über den Auslauf 7 das Dosiergerät verlässt.

Die Pulverdosiereinrichtung ist mit einer Steuerung 15 versehen. Die Steuerung 15 steht mit dem Magnetventil 4, dem Durchflussmengenmesser 5 und dem Motor 12 sowie mit Sensoren 16, 17 und 18 in Verbindung. Die Sensoren 16, 17 und 18 überwachen an unterschiedlichen Stellen die Bevorratung und Abgabe des Pulvers. So kontrolliert der erste Sensor 16 den Füllstand des Pulvers im Vorratsbehälter 10. Der zweite Sensor 17 ist im Auslass 13 angeordnet und überwacht die Menge des von der Extruderschnecke in den Auslass geförderten Pulvers. Der dritte Sensor 18 befindet sich in der Mischkammer 9 und stellt eine Überlaufkontrolle bereit.

Die Sensoren 16, 17 und 18 überwachen die Pulverdosiereinrichtung. Der im Bereich des Vorratsbehälters 10 angeordnete Sensor 16 überwacht den Füllstand des Pulvervorrats. Im Ausführungsbeispiel handelt es sich um einen kapazitiv arbeitenden Sensor. Er spricht an, wenn keine für den einwandfreien Betrieb der Dosiereinrichtung ausreichende Menge an Pulver mehr vorhanden ist. In diesem Fall sperrt die Steuerung 15 den Wasserzulauf 1 und hält den Motor 12 an. Eine Wiederaufnahme des Betriebs ist erst bei wieder gefülltem Vorratsbehälter möglich. Darüber hinaus ist ein - nicht dargestellter - Sensor vorgesehen, der das Vorhandensein des Vorratsbehälters 10 detektiert. Hierbei kann es sich um einen einfachen Taster handelt.

Bei dem zweiten Sensor 17 kann es sich zum Beispiel um eine Lichtschranke handeln. Der Sensor 17 meldet, wenn während eines Pulverförderungsvorgangs kein Pulver in den Auslass 13 gelangt. Auch in diesem Fall sperrt die Steuerung 15 den Wasserzulauf 1 und hält den Motor 12 an. Der dritte Sensor 18 spricht bei Erreichen des Höchstfüllstandes in der Mischkammer 9 an, so dass die Steuerung 15 den Wasserzulauf 1 sperrt. Der Höchstfüllstand kann zum Beispiel im Falle einer Verstopfung erreicht werden; durch das Absperren des Wasserzulaufs 1 wird ein Überlaufen verhindert. Im Ausführungsbeispiel handelt es sich bei dem Sensor 18 einen konduktiv messenden Sensor. Andere Arten der Messung sind ebenfalls möglich.

Bei der Benutzung der erfindungsgemäßen Vorrichtung wird beispielsweise mittels einer Betätigungseinrichtung, bei der es sich um eine übliche einer Start-/ Stop-Taste handeln kann, ausgelöst. Hierdurch wird zunächst eine Prüfung der Vorrichtung mit Hilfe der Sensoren vorgenommen. Es wird geprüft, ob der Vorratsbehälter 10 vorhanden ist und ob ein ausreichender Füllstand im Vorratsbehälter 10 vorliegt. Ist dies der Fall, öffnet das Magnetventil 4, so dass eine definierte Wassermenge durch den Zulauf 1 in die Vorrichtung eintritt. Das Wasser passiert den Schmutzfänger 2, den Mengenregler 3, das Magnetventil 4 sowie den Durchflussmengensensor 5 und läuft in den Wasservorlaufbehälter 6 ein. Das einlaufende Wasser fällt über die freie Strecke (bspw. gebildet von einem System-Trenner Typ CA) und tritt durch den Auslauf aus dem Behälter 6 aus. Vorher, nämlich bei Erreichen des Abzweigs 8, wird der Wasserstrom getrennt. Während der erste Teilstrom des Wassers über die Impfstelle 14 zum Auslauf 7 strömt, wird der zweite Teilstrom zur Mischkammer 9 geleitet.

Parallel zum Eintritt des Wassers in die Vorrichtung beginnt die Förderung des Pulvers. Dabei wird abgefragt, ob der Durchflussmengensensor 5 den Eintritt einer ausreichenden Menge Wasser in die Vorrichtung misst. Ist dies nicht der Fall, wird die Förderung gestoppt. Im Falle der Förderung wird der Motor 12 durch die Steuerung 15 angesteuert, so dass die Extruderschnecke 11 in Gang gesetzt wird. Dadurch wird Desinfektions- und/oder Reinigungspulver zum Auslass 13 gefördert, von wo aus das Pulver in die Mischkammer 9 gelangt. Dort wird das Pulver mit dem Wasser gemischt und von dort zur Impfstelle 14 geleitet, in der sich der zweite Teilstrom an Wasser mit dem darin befindlichen Desinfektions- und/oder Reinigungspulver mit dem ersten Teilstrom des Wassers mischt.

Das Gemisch aus dem zweiten Teilstrom an Wasser und dem darin befindlichen Desinfektions- und/oder Reinigungspulver hat auf der Strecke zwischen Mischkammer 9 und Impfstelle 14 eine Konzentration an Desinfektions- und/oder Reinigungsmittel, die über der für die Benutzung der Desinfektions- und/oder Reinigungsmittelgebrauchslösung gewünschten Konzentration liegt. Durch das Mischen mit dem ersten Teilstrom des Wassers wird am Auslauf 7 aber die gewünschte Konzentration erzielt.

Die gewünschte Konzentration an Desinfektions- und/oder Reinigungsmittel am Auslauf 7 ist von den Einsatzbedingungen und den verwendeten Mitteln abhängig. Sie wird vor der ersten Inbetriebnahme an einer - nicht dargestellten - Elektronik programmiert. In Abhängigkeit von den eingestellten Konzentrationswerten sowie in Abhängigkeit vom gemessenen Volumenstrom an Wasser lässt die Steuerung 15 mehr oder weniger Pulver von der Extruderschnecke 11 in die Mischkammer 9 fördern.

Der Fördervorgang kann dabei kontinuierlich oder diskontinuierlich erfolgen. Bei einer kontinuierlichen Förderung treibt der Motor 12 über den gesamten Zeitraum der Entnahme der Gebrauchslösung die Extruderschnecke 11 an, so dass dem zweiten Strom des Wassers dauerhaft Pulver beigemischt wird. Bei einer diskontinuierlichen Förderung treibt der Motor 12 die Extruderschnecke 11 lediglich in Intervallen an, so dass das Pulver lediglich schubartig mit dem Wasser gemischt wird.

Zum Abschluss der Entnahme der Gebrauchslösung wird der Motor 12 für einen definierten Zeitraum rückwärts betrieben, vorzugsweise für eine bis zwei Umdrehungen. Die Extruderschnecke 11 wird dadurch entgegen der Förderrichtung bewegt. Dadurch wird eine Kompression des Pulvers in der Extruderschnecke 11 und dem Behälter 10 verhindert. Gleichzeitig wird dadurch einem Verklumpen des Pulvers im Bereich des dem Auslass 13 zugewandten Endes der Extruderschnecke 11 während der Nicht-Benutzung der Vorrichtung vorgebeugt.

Darüber hinaus wird mit Hilfe der Messungen des Durchflussmengensensors 5 zum Ende eines Dosiervorgangs überwacht, dass eine ausreichende Wassermenge nach Beendigung der Pulverdosierung durch die Vorrichtung strömt, um zu gewährleisten, dass auch geringste Pulverrückstände ausgespült werden.

Der Vorratsbehälter 10 kann mit einer - nicht dargestellten - Einrichtung zum Bewegen des Pulvers versehen sein, um das Nachrutschen des Pulvers in Richtung der Extruderschnecke 11 zu verbessern. Die Einrichtung kann in einfacher Weise von einem an einer horizontal ausgerichteten Achse angeordneten Zahnrad gebildet sein, dessen Zähne mit der Extruderschnecke 11 kämmen. Folglich wird das Zahnrad in Bewegung gesetzt, wenn der Motor 12 und damit die Extruderschnecke 11 in Gang gesetzt werden.

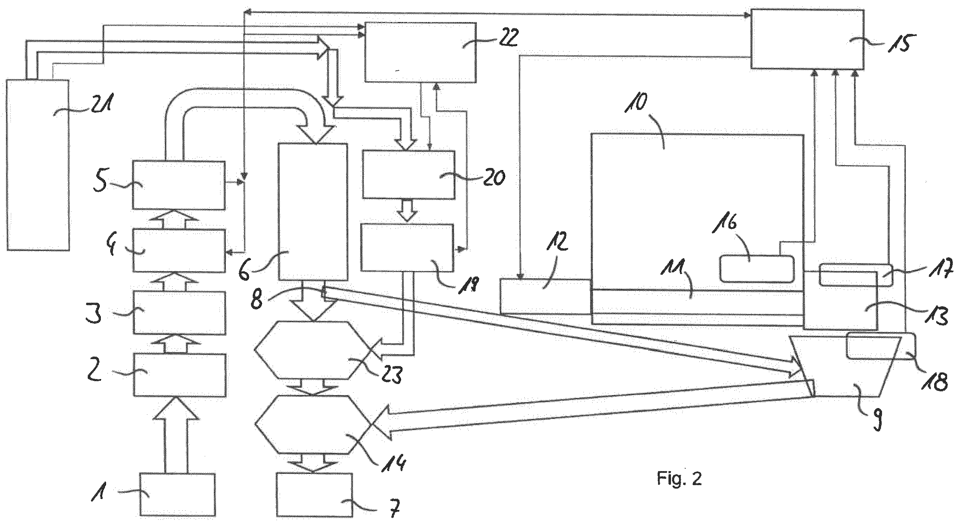

Die als Ausführungsbeispiel in Figur 2 gewählte Vorrichtung zum Dosieren von Desinfektions- und/oder Reinigungsmitteln stimmt weitgehend mit dem oben beschriebenen Ausführungsbeispiel nach Figur 1 überein. Sie weist aber zusätzlich eine Dosiereinrichtung für flüssige Desinfektions- und/oder Reinigungskonzentrate auf.

Die Dosiereinrichtung für flüssige Desinfektions- und/oder Reinigungskonzentrate umfasst eine Durchflussmessung 19 und eine Dosierpumpe 20. Die Dosierpumpe 20 ist vorzugsweise frequenzgesteuert. Dies bietet im Verhältnis zum üblichen An- und Ausschalten der Pumpe den Vorteil, dass eine homogene Lösung kontinuierlich förderbar ist, da ständig eine gleich bleibende Menge Konzentrat gefördert wird. Die Dosiereinrichtung umfasst weiterhin einen Konzentratbehälter 21 mit Reinigungsmittel und/oder Desinfektionsmittel. Zum Fördern der Mittel kann eine - nicht dargestellte - Sauglanze Anwendung finden, die in den Konzentratbehälter 21 reicht. Der Konzentratbehälter 21 ist ebenso wie die Dosierpumpe 20 und die Durchflussmessung 19 mit einem Steuergerät 22 verbunden.

Das Steuergerät 22 dient zur Einschaltung und Einstellung der Dosiereinrichtung. Es kann das Konzentrat eingestellt werden. Auch der Höchstfüllstand im Wasservorlaufbehälter 6 kann überwacht werden und gegebenenfalls wird die Dosiereinrichtung über das Magnetventil 4 abgeschaltet. Auch bei einem Konzentratmangel erfolgt eine Abschaltung. Das Steuergerät 22 erkennt eine Benutzung

Bei der Benutzung der erfindungsgemäßen Vorrichtung gemäß dem Ausführungsbeispiel nach Figur 2 wird die oben bereits erwähnte Betätigungseinrichtung ausgelöst, wodurch Wasser in der beschriebenen Weise durch den Zulauf 1 in die Vorrichtung eintritt und den Schmutzfänger 2, den Mengenregler 3, das Magnetventil 4, den Durchflussmengensensor 5 sowie Wasservorlaufbehälter 6 passiert. Es tritt durch den Auslauf aus dem Behälter 6 aus. Bei Erreichen des Abzweigs 8 wird der Wasserstrom getrennt, wenn die Herstellung einer Gebrauchslösung aus pulverförmigem Reinigungs- und/oder Desinfektionsmittelkonzentrat gewünscht ist. Während der erste Teilstrom des Wassers über die Impfstelle 14 und eine zweite Impfstelle 23 zum Auslauf 7 strömt, wird der zweite Teilstrom zur Mischkammer 9 geleitet und an der ersten Impfstelle 14 dem ersten Strom des Wassers wieder zugeführt. Über den Auslauf 7 verlässt das Wasser die Vorrichtung.

Bei der Herstellung einer Gebrauchslösung aus flüssigem Reinigungs- und/oder Desinfektionsmittelkonzentrat stellt der Durchflussmengensensor 5 die Menge an durchfließendem Wasser fest und leitet diesen Messwert an das Steuergerät 22 weiter. Das Steuergerät 22 setzt daraufhin die Dosierpumpe 20 in Gang, so dass Konzentrat aus dem Konzentratbehälter 21 gefördert wird. Über die Dosierpumpe 20 und die Durchflussmessung 19 gelangt das flüssige Reinigungs- und/oder Desinfektionsmittelkonzentrat zu der zweiten Impfstelle 23, an der das Konzentrat dem ersten Strom des Wassers beigemischt wird. Das so erstellte Gemisch passiert die erste Impfstelle 14, an der der zweite Strom des Wassers zugeführt wird. Das Gemisch aus Wasser und Konzentrat hat nach der ersten Impfstelle 14 das gewünschte Mischungsverhältnis, so dass die Gebrauchslösung am Auslauf 7 entnommen werden kann.

Selbstverständlich ist es beim Ausführungsbeispiel nach Figur 2 auch möglich, eine Kombination von pulverförmigem Reinigungs- und/oder Desinfektionsmittel und flüssigem Reinigungs- und/oder Desinfektionsmittel zu verwenden. In diesem Fall werden die Fördervorgänge durch die Steuerung 15 und die Steuergerät 22 ausgelöst, sobald eine Entnahme detektiert wird.

Die Vorrichtung beinhaltet eine Zeitmessung, die beispielsweise in die Steuerung 15 integriert sein kann. In Kombination mit der Verbindung mit dem Durchflussmengensensor 5 kann die Vorrichtung daher feststellen, welcher Zeitraum zwischen der letzten Entnahme von Wasser und / oder von Desinfektionsmittel vergangen ist. Sie kann daher selbsttätig auslösen, wenn der Zeitraum zwischen zwei Wasserentnahmen eine bestimmte, voreingestellte Dauer überschreitet, um einer möglichen Verkeimung in der Vorrichtung vorzubeugen.

Im Falle einer solchen automatischen Auslösung wird eine Spülung verkeimtem Wasser verhindert. Die im Ausführungsbeispiel dargestellte Vorrichtung ist dabei derart aufgebaut, dass bei einem automatischen Auslösen Wasser durch den Zulauf 1 in die Vorrichtung einströmt und den Schmutzfänger 2, den Mengenregler 3, das Magnetventil 4, den Durchflussmengensensor 5, den Wasservorlaufbehälter 6 sowie die Impfstellen 14 und 23 passiert und über den Auslauf 7 die Vorrichtung verlässt. An dem Abzweig 8 wird in diesem Fall immer der Wasserstrom getrennt, um eine zwangsweise Durchspülung auch der Mischkammer 9 sowie der zugehörigen Leitungen zu gewährleisten und einer Verkeimung auch dieses Abschnitts der Vorrichtung vorzubeugen. Eine Entnahme von pulverförmigem Reinigungs- und/oder Desinfektionsmittelkonzentrat erfolgt in diesem Fall nicht.

Im Ausführungsbeispiel nach Figur 2 erhält im Falle einer solchen automatischen Spülung auch das Steuergerät 22 einen Impuls. Das Steuergerät 22 setzt daraufhin die Dosierpumpe 20 in Gang, so dass Konzentrat aus dem Konzentratbehälter 21 gefördert wird. Über die Dosierpumpe 20 und die Durchflussmessung 19 gelangt das flüssige Reinigungs- und/oder Desinfektionsmittelkonzentrat über die zweite Impfstelle 23 in den Strom des Wassers und verlässt nach Passieren der ersten Impfstelle 14, an der der zweite Strom des Wassers zugeführt wird, die Vorrichtung. Auf diese Weise ist auch eine Verkeimung der Leitungen und Aggregate in der Dosiereinrichtung für flüssige Desinfektions- und/oder Reinigungskonzentrate verhindert.

Mit Hilfe der Messungen des Durchflussmengensensors 5 wird bei einer automatischen Spülung die Menge an durchfließendem Wasser überwacht. Nach Durchlauf einer vorbestimmten Menge wird der automatische Spülvorgang beendet. Die Beendigung erfolgt nach Durchlauf einer ausreichenden Menge von Wasser und im Ausführungsbeispiel nach Figur 2 auch von Desinfektionsmittel, so dass das möglicherweise mit Keimen belastete Stagnationswasser aus dem Wasserzulauf 1 sowie der gesamten Vorrichtung abgelaufen ist. Die im Anschluss an diese Signalisierung entnommene Desinfektionsgebrauchsmittellösung ist aus Frischwasser hergestellt und daher nicht belastet, so dass der Gebrauch der Lösung unbedenklich ist.

Mit der Vorrichtung nach der vorliegenden Erfindung ist die Möglichkeit geschaffen, das Bedienpersonal rechtzeitig vorder Entstehung von Keimen in den Leitungen und Geräten bis zur Entnahmestelle zu warnen, in dem die Mess- und Steuereinrichtung 12 detektiert, in welchen Abständen Wasser entnommen wird. Bei Überschreiten eines bestimmten Zeitraums des Nichtgebrauchs erfolgt eine Alarmierung, die den jeweiligen Bediener zur Ergreifung erforderlicher Maßnahmen auffordert.

## Patentansprüche

1. Vorrichtung zum Dosieren von Desinfektions- und/oder Reinigungsmitteln zur Erstellung von Gebrauchslösungen umfassend einen Wasserzulauf (1), ein Magnetventil (4), einen Durchflussmengensensor (5), einen Wasservorlaufbehälter (6) sowie einen Auslauf (7), **dadurch gekennzeichnet, dass** eine Pulverdosiereinrichtung vorgesehen ist, die als Bestandteil eine Mischkammer (9) hat, und dass stromabwärts des Wasservorlaufbehälters (6) ein Abzweig (8) mit der Mischkammer (9) verbunden ist.

2. Vorrichtung nach Anspruch 1, **dadurch gekennzeichnet, dass** die Pulverdosiereinrichtung einen Vorratsbehälter (10) für ein Desinfektions- und/oder Reinigungspulver umfasst.

3. Vorrichtung nach Anspruch 2, **dadurch gekennzeichnet, dass** in dem Vorratsbehälter (10) bodenseitig eine Extruderschnecke (11) angeordnet ist, die von einem Motor (12) angetrieben ist.

4. Vorrichtung nach Anspruch 3, **dadurch gekennzeichnet, dass** der Motor (12) einen Vorwärts- und einen Rückwärtslauf hat.

5. Vorrichtung nach einem der Ansprüche 2 bis 4, **dadurch gekennzeichnet, dass** an dem Vorratsbehälter (10) ein Auslass (13) vorgesehen, der über der Mischkammer (9) positioniert ist.

6. Vorrichtung nach einem der vorgenannten Ansprüche, **dadurch gekennzeichnet, dass** die Mischkammer (9) mit einer Impfstelle (14) verbunden ist.

7. Vorrichtung nach einem der Ansprüche 2 bis 6, **dadurch gekennzeichnet, dass** der Vorratsbehälter (10) mit einer Einrichtung zum Bewegen des Pulvers versehen ist.

8. Verfahren zum Dosieren von pulverförmigen Desinfektions- und/oder Reinigungsmitteln zur Erstellung von Gebrauchslösungen umfassend folgende Schritte:
• Eintreten von Wasser durch einen Zulauf (1)
• Passieren eines Magnetventils (4) und eines Durchflussmengensensors (5)
• Leiten mindestens eines Teils des Wassers zu einer Mischkammer (9)
• Messen des Volumenstroms durch den Durchflussmengensensor (5)
• Weiterleiten des Messergebnisses an eine Steuerung (15)
• Fördern des pulverförmigen Desinfektions- und/oder Reinigungsmittels in die Mischkammer (9)
• Mischen des pulverförmigen Desinfektions- und/oder Reinigungsmittels mit dem Wasser
• Weiterleitung des Wasser-Desinfektions- und/oder Reinigungspulver-Gemisches zu einem Auslass (13).

9. Verfahren nach Anspruch 8, **dadurch gekennzeichnet, dass** das Desinfektions- und/oder Reinigungsmittel mit einer Extruderschnecke (11) in die Mischkammer (9) gefördert wird.

10. Verfahren nach Anspruch 8 oder 9, **dadurch gekennzeichnet, dass** das Desinfektions- und/oder Reinigungsmittel kontinuierlich gefördert wird.

11. Verfahren nach Anspruch 8 oder 9, **dadurch gekennzeichnet, dass** das Desinfektions- und/oder Reinigungsmittel diskontinuierlich gefördert wird.

12. Verfahren nach einem der Ansprüche 8 bis 11, **dadurch gekennzeichnet, dass** zum Abschluss der Förderung der Motor (12) rückwärts betrieben wird.

## Claims

1. Device for dosing of disinfectants and/or cleaning agents for the preparation of ready-to-use solutions comprising a water inlet (1), a magnetic valve (4), a flow rate sensor (5), a water feed tank (6) as well as an outlet (7), **characterised in that** a powder dosing device is provided, which has as a component a mixing chamber (9) and that downstream of the water feed tank (6) a branch (8) is connected to the mixing chamber (9).

2. Device according to claim 1, **characterised in that** the powder dosing device comprises a storage container (10) for a disinfectant and/or cleaning powder.

3. Device according to claim 2, **characterised in that** an extruder screw (11), which is driven by a motor (12), is arranged at the bottom in the storage container (10).

4. Device according to claim 3, **characterised in that** the motor (12) has a forward and a reverse run.

5. Device according to one of claims 2 to 4, **characterised in that** an outlet (13), which is positioned above the mixing chamber (9), is provided on the storage container (10).

6. Device according to one of the previous claims, **characterised in that** the mixing chamber (9) is connected to an injection point (14).

7. Device according to one of claims 2 to 6, **characterised in that** the storage container (10) is provided with a device for moving the powder.

8. Method for dosing of powdery disinfectants and/or cleaning agents for the preparation of ready-to-use solutions comprising the following steps:
• entry of water through an inlet (1)
• passing a magnetic valve (4) and a flow rate sensor (5)
• conducting at least a part of the water to a mixing chamber (9)
• measuring the volume flow rate by the flow rate sensor (5)
• forwarding the measurement result to a control (15)
• conveying the powdery disinfectant and/or cleaning agent to the mixing chamber (9)
• mixing the powdery disinfectant and/or cleaning agent with the water
• forwarding the mixture of water and disinfectant and/or cleaning agent to an outlet (13).

9. Method according to claim 8, **characterised in that** the disinfectant and/or cleaning agent is conveyed into the mixing chamber (9) by an extruder screw (11).

10. Method according to claim 8 or 9, **characterised in that** the disinfectant and/or cleaning agent is continuously conveyed.

11. Method according to claim 8 or 9, **characterised in that** the disinfectant and/or cleaning agent is discontinuously conveyed.

12. Method according to one of claim 8 to 11, **characterised in that** the motor (12) is operated in reverse in order to complete conveying.

## Revendications

1. Dispositif de dosage d'agents de désinfection et/ou d'agents de nettoyage pour l'élaboration de solutions à utiliser comprenant une arrivée d'eau (1), une électrovanne (4), un capteur débitmétrique (5), un réservoir (6) sur le circuit aller d'eau ainsi qu'une sortie d'eau (7), **caractérisé en ce qu'**est prévu un dispositif de dosage de poudre composé d'un compartiment mélangeur (9), et qu'en aval du réservoir (6) sur le circuit aller un piquage (8) est relié au compartiment mélangeur (9).

2. Dispositif selon la revendication 1, **caractérisé en ce que** le dispositif de dosage de poudre comprend un réservoir de stockage (10) pour une poudre de désinfection et/ou de nettoyage.

3. Dispositif selon la revendication 2, **caractérisé en ce que** dans le réservoir de stockage (10) est disposée, du côté du fond de ce dernier, une vis sans fin d'extrusion (11) entraînée par un moteur (12).

4. Dispositif selon la revendication 3, **caractérisé en ce que** le moteur (12) présente une marche avant et une marche arrière.

5. Dispositif selon l'une des revendications 2 à 4, **caractérisé en ce que** contre le réservoir de stockage (10) est prévu un orifice de sortie (13) positionné au-dessus du compartiment mélangeur (9).

6. Dispositif selon l'une des revendications précédentes, **caractérisé en ce que** le compartiment mélangeur (9) est relié à un poste d'inoculation (14).

7. Dispositif selon l'une des revendications 2 à 6, **caractérisé en ce que** le réservoir de stockage (10) est muni d'un dispositif permettant de déplacer la poudre.

8. Procédé pour le dosage d'agents de désinfection et/ou d'agents de nettoyage pulvérulents pour l'élaboration de solutions à utiliser, comprenant les étapes suivantes:
• Pénétration de l'eau par une arrivée d'eau (1)
• Traversée d'une électrovanne (4) et d'un capteur débitmétrique (5)
• Envoi d'au moins une partie de l'eau vers un compartiment mélangeur (9)
• Mesure du débit volumique par le capteur débitmétrique (5)
• Communication du résultat de mesure à une commande (15)
• Transport de l'agent de désinfection et/ou de l'agent de nettoyage pulvérulent vers le compartiment mélangeur (9)
• Mélange de l'agent de désinfection et/ou de l'agent de nettoyage avec l'eau
• Poursuite de l'acheminement du mélange eau/poudre désinfectante et/ou poudre nettoyante vers un orifice de sortie (13).

9. Procédé selon la revendication 8, **caractérisé en ce que** l'agent de désinfection et/ou l'agent de nettoyage est transporté jusque dans le compartiment mélangeur (9) au moyen d'une vis sans fin d'extrusion (11).

10. Procédé selon la revendication 8 ou 9, **caractérisé en ce que** l'agent de désinfection et/ou l'agent de nettoyage est transporté en permanence.

11. Procédé selon la revendication 8 ou 9, **caractérisé en ce que** l'agent de désinfection et/ou l'agent de nettoyage est transporté de façon discontinue.

12. Procédé selon l'une des revendications 8 à 11, **caractérisé en ce qu'**à la fin du transport le moteur (12) est exploité en marche arrière.
